# EUROPÄISCHE PATENTSCHRIFT

(11) **EP 0 643 953 B1**
(45) Veröffentlichungstag und Bekanntmachung des Hinweises auf die Patenterteilung: **16.08.2001**
(21) Anmeldenummer: 94114066.7
(22) Anmeldetag: 08.09.1994
(51) Int. Cl.: A61F 2/48, A61F 5/48

(54) **Implantierbare Vorrichtung zum Steuern der Schliessmuskel**
Implant for control of sphincters
Dispositif implantable pour la commande des sphincters

(30) Priorität: 17.09.1993 DE 4331658
(43) Veröffentlichungstag der Anmeldung: 22.03.1995
(73) Patentinhaber: ESKA medical GmbH & Co., D-23556 Lübeck (DE)
(72) Erfinder: Grundei, Hans, Dr., D-23558 Lübeck (DE); Ahlers, Olaf, D-22043 Hamburg (DE)
(74) Vertreter: Wilcken, Thomas, Dipl.-Ing.

(56) Entgegenhaltungen:
- EP-A- 0 409 592
- DE-A- 2 438 691
- US-A- 4 587 954
- US-A- 4 828 544
- US-A- 4 961 725
- US-A- 5 123 428

## Beschreibung

Die Erfindung betrifft eine implantierbare Vorrichtung nach dem Oberbegriff des Anspruchs 1.

Implantierbare Vorrichtungen zum Öffnen und Verschließen von rohrförmigen Körperorganen werden insbesondere in Form von Prothesen zur Behandlung der Harninkontinenz benutzt und sind auch unter der Bezeichnung "Sphinkter" bekannt. Dabei werden Verschlüsse benutzt, die mechanisch auf die Harnröhre einwirken und beim Versagen des natürlichen Schließmuskels dessen Funktion übernehmen. Solche Verschlüsse sind in vielerlei Varianten bekannt. Aus der CH-PS 463 015 ist eine implantierbare Vorrichtung zum wahlweisen Öffnen und Verschließen der Harnröhre bekannt, bei der gegeneinander bewegbare Klemmteile zum Verschließen der Harnröhre vorgesehen sind. Die Klemmteile stehen unter der Einwirkung einer Feder und eines Magneten, so daß sie von außen betätigbar sind. Bekannt ist auch eine Prothese für die männliche Harninkontinenz (DE-GU 79 29 052), bei der eine die Harnröhre umschließende Manschette durch Druckausübung einen Verschluß der Harnröhre ermöglicht. Auch bei bekannten Prothesen nach der EP 144 699 B1 umschließt jeweils eine Manschette die Harnröhre von außen, wobei die Erzielung der Öffnungs- bzw. Schließstellung durch mechanischen Druck von außen oder auch durch Flüssigkeitsdruck erfolgt. Dazu ist eine implantierte Pumpe mit einem implantierten Flüssigkeitsreservoir vorhanden.

Bei einer implantierbaren Vorrichtung nach dem Oberbegriff des Anspruchs 1 (US-A-4 828 544) wird ein Blutgefäß von einer Manschette umschlossen, die einen Blähkörper in ihrem Inneren aufweist, so daß das Blutgefäß nach Füllung des Blähkörpers von außen eingeengt und abgeschnürt werden kann.

Die Einengung und Abschnürung der Harnröhre ist aus EP-A-0 409 592 bekannt. Auch hierbei wird eine Muffe benutzt, die über Pumpen betätigtes Fluid verengt bzw. gelöst werden kann.

In allen vorgenannten Fällen wird in bekannter Weise die Harnröhre durch Druck von außen wahlweise verschlossen oder geöffnet. Selbst dann aber, wenn als Druckorgan sehr weiche und schonend einwirkende Manschetten verwendet werden, tritt nach verhältnismäßig kurzer Zeit im Bereich des Verschlußorgans eine nekrotische Schädigung der Harnröhre bzw. ihres umgebenden, gut durchbluteten Gewebes auf. Der Verschluß muß dann entfernt werden. Eine Dauerlösung des Inkontinenzproblems oder auch nur eine Lösung für wenigstens eine längere Zeit läßt sich demnach mit solchen Vorrichtungen nicht erreichen. Entsprechendes gilt auch für den Fall, daß andere Körperorgane wahlweise geöffnet oder verschlossen werden sollen, beispielsweise Blutgefäße.

Aus der DE-PS 41 35 502 ist auch bereits ein Inkontinenzventil bekannt, das von einem in seiner ganzen Länge in die Harnröhre einzuführenden, länglichen Ventilkörper mit Längsbohrung gebildet ist. Der Ventilkörper besitzt ein offenes und ein verschließbares Ende mit einem Ventilkopf. Der Harn wird an der Außenseite des Ventils zumindest einer radial angeordneten Verbindungsöffnung in die Längsbohrung geführt, wobei die Verbindungsöffnung über eine vom distalen Ende aus betätigbare Absperreinrichtung wahlweise freigegeben oder verschlossen werden kann. Dieses bekannte Ventil vermeidet zwar die Nachteile der oben erläuterten Ventile, bei denen die Harnröhre von außen zusammengepreßt wird, aber es verbleiben weitere Probleme. Insbesondere besteht nicht die Möglichkeit, einen Katheter durch das bekannte Ventil zu schieben, was in Notfällen und für Untersuchungen nötig ist. Von diesem bekannten Ventil wird im Oberbegriff des Anspruchs 1 ausgegangen.

Der Erfindung liegt demgemäß die Aufgabe zugrunde, eine implantierbare Vorrichtung nach dem Oberbegriff des Anspruchs 1 zu schaffen, die nicht nur ein sicheres Öffnen und Verschließen des Körperorgans ermöglicht, sondern darüber hinaus weiterhin das Ein- bzw. Durchführen eines Katheters ermöglicht und außerdem Bewegungen des Organs elastisch folgen kann. Die Lösung der Aufgabe ist im Patentanspruch 1 gekennzeichnet. Weiterbildungen der Erfindung sind Gegenstand der Unteransprüche.

Der Blähkörper wird zweckmäßig durch eine Blase gebildet, in die ein Schlauch zur Zuführung des Fluid mündet. Im allgemeinen ist es zweckmäßig, als Fluid eine Flüssigkeit, beispielsweise physiologische Kochsalzlösung, zu verwenden, weil Flüssigkeiten praktisch nicht kompressibel sind. Gegebenenfalls läßt sich aber auch ein Gas einsetzen.

Die Blase wird mit Vorteil durch ein an beiden Seiten verschlossenes Innenschlauchstück gebildet, dessen Außenfläche über seine Länge und auf einem Teil seines Umfangs mit der Innenfläche des Schlauchabschnitts verbunden, beispielsweise verklebt oder verschweißt, ist. Der Verschluß des Innenschlauchstücks kann mit Vorteil durch Flachdrücken und Verkleben der Enden erfolgen, wobei dann das Innenschlauchstück mit einer Seite der flachgedrückten Enden und dem zwischenliegenden Teil des Umfangs mit der Innenfläche des Schlauchabschnitts verbunden ist. Die flachgedrückten Enden laufen dabei zweckmäßig keilförmig aus, so daß sie ohne Stufe in die Wand des Schlauchabschnitts übergehen. Ein Katheter kann dann ohne anzustoßen durchgeschoben werden.

Der Ventilkörper besteht aus elastischem Kunststoff, vorzugsweise Siliconkautschuk. Er wird zweckmäßig mit einer Gewebeeinlage oder -auflage versehen, die eine Durchmesservergrößerung des Schlauchabschnitts begrenzt. Auf diese Weise wird vermieden, daß die Harnröhre bzw. das jeweilige Körperorgan zu sehr gedehnt werden kann, auch wenn der Innendruck des Blähkörpers stärker ansteigt.

Das Fluid in Form einer Flüssigkeit läßt sich zweckmäßig mittels einer implantierbaren, manuell bedienbaren Pumpeinrichtung über einen Schlauch in den Blähkörper fördern. An die Pumpeinrichtung ist mit Vorteil ein ebenfalls implantierbares Vorratsgefäß angeschlossen, und die Pumpeinrichtung weist ein in der Zuleitung zum Vorratsgefäß liegendes, manuell von außen betätigbares Rückschlagventil auf, derart, daß bei Betätigung der Pumpeinrichtung Flüssigkeit aus dem Blähkörper abgesaugt und in das Vorratsgefäß gefördert wird. Der Blähkörper fällt dann unter Freigabe des Durchflusses durch den Schlauchabschnitt zusammen. Bei Betätigung des Rückschlagventils strömt Flüssigkeit unter dem Einfluß des Drucks im Vorratsgefäß in den Blähkörper und sperrt den Durchfluß im Schlauchabschnitt. Dabei soll die Flüssigkeitsmenge in dem aus dem Vorratsbehälter, dem Blähkörper, der Pumpeinrichtung und den Verbindungsschläuchen bestehenden System so bemessen sein, daß ein bestimmter, für ein sicheres Sperren des Durchflusses durch den Ventilkörper ausreichender Systemdruck nicht überschritten wird. Auf diese Weise wird zum einen vermieden, daß unnötig hohe Drücke im System auftreten, und zum anderen eine Notventilfunktion verwirklicht. Wenn nämlich beispielsweise der Druck in der Harnblase über einen normalen Wert ansteigt, sperrt der Blähkörper den Durchfluß durch den Ventilkörper nicht mehr vollständig, so daß der Druckanstieg begrenzt wird und im Notfall ein Abfluß erfolgen kann.

Nachfolgend wird ein Ausführungsbeispiel der Erfindung anhand der Zeichnungen beschrieben. Es zeigen:
- Fig. 1: schematisch eine implantierbare Vorrichtung in Form eines Inkontinenzventils mit einer Pumpeinrichtung und einem Vorratskörper, implantiert bei einem Manne;
- Fig. 2: einen Längsschnitt durch den Ventilkörper nach Fig. 1;
- Fig. 3: eine Aufsicht auf den Ventilkörper nach Fig. 2;
- Fig. 4: einen Querschnitt durch den Ventilkörper nach Fig. 2 und 3.

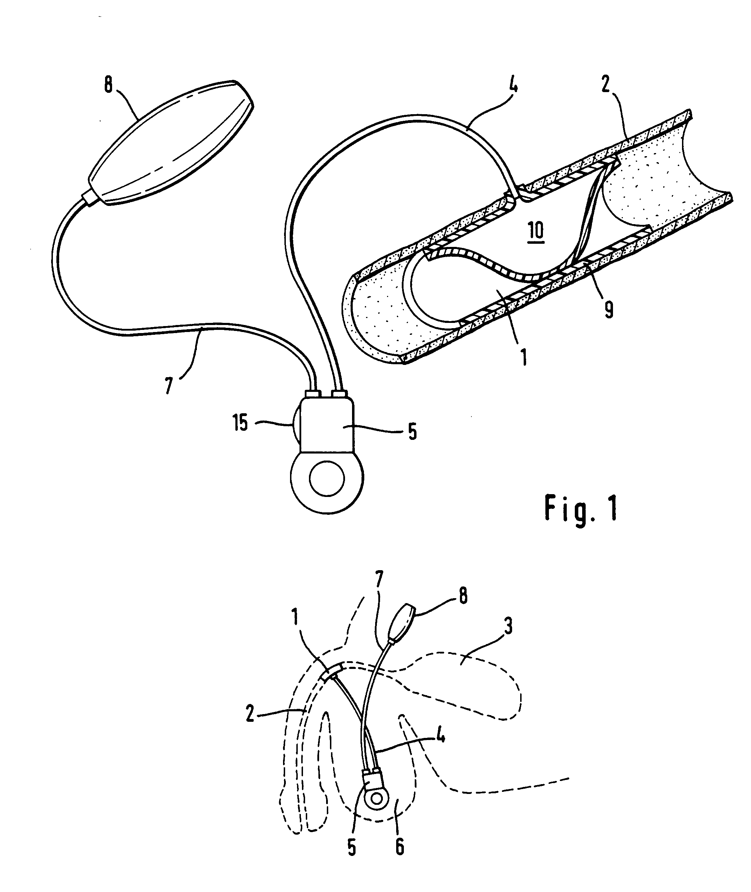

In Fig. 1 ist im unteren Teil schematisch dargestellt, wie eine Vorrichtung nach der Erfindung im menschlichen Körper implantiert ist, im dargestellten Beispiel bei einem Mann. Der Ventilkörper 1 ist in die Harnröhre 2 in einem Bereich nahe der Blase 3 eingesetzt. Bei der Implantation wird die Harnröhre an der gewählten Stelle geschlitzt, so daß der Ventilkörper 1 eingeschoben werden kann. Zum Ventilkörper 1 führt ein Schlauch 4 von einer Pumpeinrichtung 5, die in das Skrotum 6 so eingefügt ist, daß sie von außen tastbar und betätigbar ist. An die Pumpeinrichtung 5 ist über einen weiteren Schlauch 7 ein Vorratsbehälter 8 angeschlossen, der an geeigneter Stelle unter der Bauchdecke liegt.

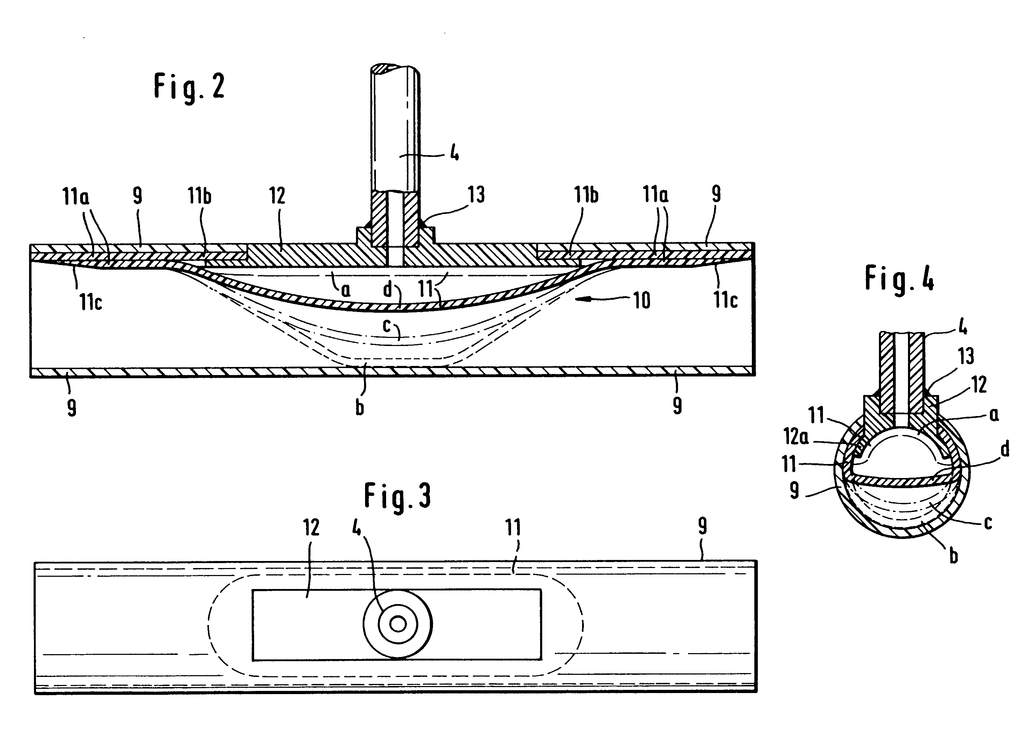

Wie im oberen Teil von Fig. 1 vergrößert gezeigt ist, weist der in der Harnröhre 2 angeordnete Ventilkörper 1 einen Schlauchabschnitt 9 auf, in dem sich ein Blähkörper 10 befindet, der im dargestellten Zustand aufgebläht ist und den Schlauchabschnitt 9 und damit die Harnröhre 2 verschließt. Das Aufblähen erfolgt durch eine Flüssigkeit, die mittels der Pumpeinrichtung 5 über den Schlauch 4 gefördert wird. Auf die Funktion der Pumpeinrichtung 5, die Betätigung des Ventilkörpers 1 und die Druckverhältnisse im System wird nachfolgend noch genauer eingegangen. Zunächst soll jedoch der Ventilkörper 1 anhand der Fig. 2 bis 4 genauer beschrieben werden.

Der Ventilkörper 1, dessen Teile aus Siliconkautschuk hergestellt und durch Verkleben miteinander verbunden sind, weist einen Schlauchabschnitt 9 auf, dessen Länge und Durchmesser an den jeweiligen Verwendungszweck anzupassen ist. Bei einer Verwendung als Inkontinenzventil und Implantation in die Harnröhre kann je nach den anatomischen Verhältnissen der Außendurchmesser im Bereich zwischen 6 und 10 mm und die Länge im Bereich zwischen 20 und 80 mm, vorzugsweise bei etwa 50 mm, liegen. Die Wandstärke beträgt beispielsweise etwa 0,5 mm.

Im Innern des Schlauchabschnitts 9 ist der generell mit 10 bezeichnete Blähkörper angeordnet, der aus einem Innenschlauchstück 11 gebildet ist. An seinen beiden Enden 11a (Fig. 2) ist das Innenschlauchstück 11 durch Zusammendrücken und Verkleben verschlossen. Außerdem ist das Innenschlauchstück 11 in seinem in den Figuren oberen Umfangsteil 11b sowie mit dem oberen Teil der Innenschlauchenden 11a mit dem oberen Teil des Schlauchabschnitts 9 verbunden, beispielsweise verklebt. Eine Abflachung 11c an beiden Innenschlauchenden 11a stellt einen stufenlosen Übergang sicher.

Etwa im mittleren Bereich der miteinander verbundenen Teile des Innenschlauchstücks 11 und des Schlauchabschnitts 9 ist ein Flanschanschlußstück eingesetzt, das durch eine angepaßte Öffnung der beiden Schlauchwände aus dem Inneren des Blähkörpers 10 nach außen führt und dem Anschluß des Schlauchs 4 (Fig. 1) dient. Zur sicheren Abdichtung wird der Schlauch 4 mit dem Flanschanschlußstück 12 verschweißt oder verklebt, wie schematisch bei 13 gezeigt. Das sichere und dichte Verbinden des Flanschanschlußstücks 12 wird unterstützt durch Fortsätze 12a, die die Wände der Schläuche 9, 11 hintergreifen und dort ebenfalls verklebt sind.

In den Fig. 2 und 3 sind mehrere Positionen dargestellt, die der nicht verklebte Wandteil des Innenschlauchstücks 11 einnehmen kann. In der Position a liegt die Wand am Flanschanschlußstück 12 an. Das Innenschlauchstück 11 ist also insgesamt kollabiert, und zwar dadurch, daß mittels der Pumpe 5 über den Schlauch 4 die gesamte Flüssigkeit aus dem Innenschlauchstück 11 herausgesaugt und demgemäß ein Unterdruck gegenüber der Umgebung erzeugt worden ist. In dieser Position ist der Schlauchabschnitt 9 des Ventilkörpers 1 praktisch unbehindert durchgängig, so daß ein Harnabfluß bzw. im Fall einer anderen Verwendung der Abfluß einer anderen Körperflüssigkeit möglich ist. Außerdem ist der Schlauchabschnitt 9 auch für das Durchschieben eines Katheters oder eines Endoskops frei.

Die gestrichelt dargestellte Wandposition b des Innenschlauchstücks 11 stellt dagegen die geschlossene Position des Ventilkörpers 1 dar. Die Wand des Innenschlauchstücks 11 liegt dabei abdichtend an der Innenfläche des Schlauchabschnitts 9 an. Das wird erreicht, indem Flüssigkeit über den Schlauch 4 mit ausreichendem Druck in das Innenschlauchstück 11 eingeführt wird.

Die neutrale Position c stellt diejenige Lage dar, in der die Wand des Innenschlauchstücks 11 nicht durch einen Flüssigkeitsdruck beeinflußt ist, also weder in Richtung auf die Schließposition b noch in Richtung auf die Öffnungsposition a beaufschlagt ist. Diese neutrale Position c kann konstruktiv vorgewählt und demgemäß auch anders eingestellt werden. Die Position d stellt eine Zwischenlage zwischen den Positionen a und c dar.

Das aus dem Innenschlauchstück 11, der Pumpeinrichtung 5, dem Vorratsbehälter 8 und den verbindenden Schläuchen 4 und 7 bestehende System ist nach außen vollständig abgedichtet und mit einer Flüssigkeit, beispielsweise physiologischer Kochsalzlösung, blasenfrei gefüllt. Dazu können beispielsweise Steckanschlüsse (nicht gezeigt) in einem oder beiden Schläuchen 4, 7 vorgesehen sein. Der Innendruck im Flüssigkeitssystem, der sich bei Einfüllen einer bestimmten Flüssigkeitsmenge aufgrund der elastischen Wände aller Teile und insbesondere auch des Vorratsbehälters 8 einstellt, wird dabei so gewählt, daß beim Wirksamwerden des Drucks im Innenschlauchstück 11 dieses die geschlossenen Position b einnimmt. Nach der Implantation aller Teile kann zunächst bis zum Verheilen ein ungehinderter Harnabfluß mittels eines eingeschobenen Katheters sichergestellt werden. Später kann dann der Patient wunschgemäß das Ventil öffnen und schließen.

Zum Öffnen wird die Pumpeinrichtung 5 durch die Haut mit zwei Fingern betätigt. Ein Rückschlagventil (nicht gezeigt) in der Zufuhr zum Schlauch 7 sorgt dabei dafür, daß Flüssigkeit, die über den Schlauch 4 dem Innenschlauchstück 11 entnommen wird, in den Vorratsbehälter 8 gepumpt wird. Die Wand des Innenschlauchstücks 11 geht schließlich in die geöffnete Position a, wenn die gesamte Flüssigkeit aus dem Innenschlauchstück 11, also dem Blähkörper 10, herausgesaugt und in den Vorratsbehälter 8 gefördert ist. Wenn der Patient nach dem Harnabfluß den Ventilkörper wieder schließen will, drückt er auf eine seitliche Ausbeulung 15 der Pumpeinrichtung 5. Dadurch wird das innere Rückschlagventil geöffnet, so daß die im Vorratsbehälter 8 unter erhöhtem Druck stehende Flüssigkeit über den Schlauch 4 in das Innenschlauchstück 11 strömt und die geschlossene Position b einstellt.

Damit der Druck im Innenschlauchstück 11 auch bei erhöhten Werten nicht zu einer unzulässigen Aufweitung des Schlauchabschnitts 9 und damit der Harnröhre 2 führt, ist auf der Außenseite des Schlauchabschnitts 9 ein zugfestes Gewebe ("Mesh") aufgebracht, das in der Zeichnung nicht dargestellt ist.

## Patentansprüche

1. Implantierbare Vorrichtung zum wahlweisen Öffnen oder Schließen eines rohrförmigen Körperorgans, insbesondere der Harnröhre (2), mit folgenden Merkmalen:
ein Schlauchabschnitt (9);
ein Blähkörper (10), der innerhalb des Schlauchabschnittes (9) angebracht ist und durch ein Fluid aufblähbar ist, um die Schließstellung der Vorrichtung einzunehmen, bzw. entleerbar ist, um die Freigabestellung der Vorrichtung einzunehmen;
gekennzeichnet durch folgende Ausbildung:
der Schlauchabschnitt (9) weist einen Außendurchmesser auf, der zur Implantation in dem rohrförmigen Körperorgan (2) passend gewählt ist, so daß nach Implantation das rohrförmige Körperorgan (2) den Schlauchabschnitt (9) umgibt;
der Blähkörper (10) wird durch ein Innenschlauchstück (11) gebildet, das an beiden Enden (11a) verschlossen ist und das mit seiner Außenfläche über seine Länge und auf einem Teil seines Umfangs mit der Innenseite des Schlauchabschnitts (11) verbunden ist,
wobei die Enden (11a) des Innenschlauchstückes (11) flachgedrückt und verklebt sind;
der Blähkörper (10) liegt auf dem Schlauchabschnitt (9) dichtend an, um die Schließstellung zu bewirken.

2. Vorrichtung nach Anspruch 1,
dadurch gekennzeichnet,
daß die flachgedrückten Enden (11a) des inneren Schlauchstücks (11) keilförmig (11c) auslaufen, so daß sie ohne Stufe in die Wand des Schlauchabschnitts (9) übergehen.

3. Vorrichtung nach Anspruch 1 oder 2,
dadurch gekennzeichnet,
daß ein Schlauch (4) vorgesehen ist, der zur Zuführung des Fluids im mittleren Bereich des inneren Schlauchstücks (11) mündet.

4. Vorrichtung nach Anspruch 3, 4 oder 5,
dadurch gekennzeichnet,
daß im Einmündungsbereich des Zuführschlauches (4) ein Flanschanschlußstück (12) eingesetzt ist, das durch die verbundenen Schlauchwände führt, diese hintergreift und mit ihnen verklebt ist.

5. Vorrichtung nach einem der Ansprüche von 1 bis 4,
dadurch gekennzeichnet,
daß der Schlauchabschnitt (9) aus elastischem Kunststoff, vorzugsweise Siliconkautschuk, besteht und mit einer Gewebeein- oder -auflage versehen ist, die eine Durchmesservergrößerung des Schlauchabschnitts (9) begrenzt.

6. Vorrichtung nach einem der Ansprüche von 1 bis 5, gekennzeichnet durch eine implantierbare, manuell bedienbare Pumpeinrichtung (5), die zur Förderung des Fluids über einen bzw. den Schlauch (4) mit dem Blähkörper (10) verbunden ist.

7. Vorrichtung nach Anspruch 6,
dadurch gekennzeichnet,
daß an die Pumpeinrichtung (5) ein ebenfalls implantierbares Vorratsgefäß (8) angeschlossen ist, daß die Pumpeinrichtung (5) ein in der Zuleitung zum Vorratsgefäß (8) liegendes, manuell von außen betätigbares Rückschlagventil aufweist, derart, daß bei Betätigung der Pumpeinrichtung (5) Flüssigkeit aus dem Blähkörper (10) abgesaugt und in das Vorratsgefäß (8) gefördert wird und der Blähkörper (10) unter Freigabe des Durchflusses durch den Schlauchabschnitt (9) zusammenfällt, und
daß bei Betätigung des Rückschlagventils Flüssigkeit unter dem Einfluß des Drucks im Vorratsgefäß (8) in den Blähkörper (10) strömt und den Durchfluß im Schlauchabschnitt (9) sperrt.

8. Vorrichtung nach Anspruch 7,
dadurch gekennzeichnet,
daß die Flüssigkeitsmenge, die in dem aus dem Vorratsbehälter (8), dem Blähkörper (10), der Pumpeinrichtung (5) und den Verbindungsschläuchen (4, 7) bestehenden System eingefüllt ist, gerade ausreicht, den Blähkörper (10) in die Schließstellung zu bringen.

## Claims

1. An implantable device for the selective opening or closing of a tubular body organ, in particular the urethra (2), with the following features:
a tubing section (9);
an inflatable body (10) which is attached within the tubing section (9) and is inflatable by a fluid in order to assume the closure position of the device or which may be emptied in order to assume the release position of the device;
characterised by the following design,
the tubing section (9) comprises an outer diameter which is fittingly selected for implantation in the tubular body organ (2) so that after implantation the tubular body organ (2) surrounds the tubing section (9);
the inflatable body (10) is formed by an inner tubing piece (11) which at both ends (11a) is closed and which with its outer surface over its length and on a part of its periphery is connected to the inner side of the tubing section (11),
wherein the ends (11a) of the inner tubing section (11) are pressed flat and adhesed;
the inflatable body (10) sealingly bears on the tubing section (9) in order to effect the closure position.

2. A device according to claim 1, characterised in that the flatly pressed ends (11a) of the inner tubing sections (11) run out in a wedged manner (11c) so that without a step they blend into the wall of the tubing section (9).

3. A device according to claim 1 or 2, characterised in that there is provided a tubing (4) which for feeding the fluid opens out in the middle region of the inner tubing piece (11).

4. A device according to claim 3, 4 or 5, characterised in that in the opening-out region of the feed tubing (4) there is applied a flange connection piece (12) which leads through the connected tubing walls, engages behind these and is adhesed to them.

5. A device according to one of the claims 1 to 4, characterised in that the tubing section (8) consists of elastic plastic, preferably silicon rubber and is provided with a tissue inlay or overlay which limits a diameter enlargement of the tubing section (9).

6. A device according to one of the claims 1 to 5, characterised by an implantable, manually operable pump means (5) which for the delivery of the fluid via a or the tubing (4) is connected to the inflatable body (10)

7. A device according to claim 6, characterised in that to the pump means (5) there is likewise connected an implantable supply vessel (8), that the pump means (5) comprises a return valve which lies in the feed conduit to the supply vessel (8) and is externally manually actuatable, in a manner such that on actuation of the pump means (5) fluid is suctioned from the inflatable body (10) and conveyed into the supply vessel (8) and the inflatable body (10) amid the release of the through-flow through the tubing section (9) collapses and
that on actuation of the return valve fluid under the influence of the pressure in the supply vessel (8) flows into the inflatable body (10) and blocks the through-flow in the tubing section (9).

8. A device according to claim 7, characterised in that the fluid quantity which is filled into the system consisting of the supply container (8), the inflatable body (1), the pump means (5) and the connection tubings (4,7) is just sufficient to bring the inflatable body (10) into the closure position.

## Revendications

1. Dispositif implantable, destiné à l'ouverture ou à la fermeture sélective d'un organe corporel de forme tubulaire, notamment de l'urètre (2), présentant les caractéristiques suivantes:
un tronçon de tuyau souple (9),
un corps gonflable (10), qui est placé à l'intérieur du tronçon de tuyau souple (9) et peut être gonflé par un fluide, en vue d'assurer la position de fermeture du dispositif, ou respectivement vidé en vue d'assurer la position de passage libre du dispositif,
caractérisé par la configuration suivante :
le tronçon de tuyau souple (9) présente un diamètre extérieur, qui est choisi de manière ajustée pour l'implantation dans l'organe corporel (2) de forme tubulaire, de façon à ce qu'après implantation, l'organe corporel (2) de forme tubulaire entoure le tronçon de tuyau souple (9),
le corps gonflable (10) est formé par une pièce de tuyau souple intérieure (11) qui est fermée aux deux extrémités (11a), et qui, avec sa surface extérieure, le long de sa longueur et sur une partie de sa périphérie, est reliée au côté intérieur du tronçon de tuyau souple (9),
les extrémités (11a) de la pièce de tuyau souple intérieure (11) étant aplaties et collées,
et le corps gonflable (10) s'appliquant de manière étanche contre le tronçon de tuyau souple (9), en vue de produire la position de fermeture.

2. Dispositif selon la revendication 1, caractérisé en ce que les extrémités (11a) aplaties de la pièce de tuyau souple intérieure (11) se terminent en forme de coin (11c) de sorte qu'elles se raccordent sans décrochement à la paroi du tronçon de tuyau souple (9).

3. Dispositif selon la revendication 1 ou 2, caractérisé en ce qu'il est prévu une tubulure (4), qui pour l'amenée du fluide, débouche dans la zone centrale de la pièce de tuyau souple intérieure (11).

4. Dispositif selon la revendication 3, caractérisé en ce que dans la none d'embouchure de la tubulure d'amenée (4), est encastrée une pièce formant bride de raccordement (12), qui conduit à travers les parois de tuyau souple reliées, s'engage derrière celles-ci et y est collée.

5. Dispositif selon l'une des revendications 1 à 4, caractérisé en ce que le tronçon de tuyau souple (9) est réalisé en une matière plastique élastique, de préférence en caoutchouc silicone, et est pourvu d'un insert tissé ou d'une gaine tissée qui limite l'augmentation de diamètre du tronçon de tuyau souple (9).

6. Dispositif selon l'une des revendications 1 à 5, caractérisé par un mécanisme à pompe (5) implantable, manoeuvré manuellement, qui, pour le refoulement du fluide, est relié au corps gonflable (10), par l'intermédiaire d'une tubulure ou de ladite tubulure (4).

7. Dispositif selon la revendication 6, caractérisé en ce qu'au mécanisme de pompe (5) est raccordé un réservoir (8) également implantable, en ce que le mécanisme de pompe (5) comporte une soupape anti-retour se trouvant dans la conduite de liaison au réservoir (8) et pouvant être actionnée manuellement de l'extérieur, de façon telle que pour un actionnement du mécanisme de pompe (5), du liquide est aspiré du corps gonflable (10) et est refoulé dans le réservoir (8), le corps gonflable (10) s'effondrant suite à l'ouverture du passage d'écoulement à travers le tronçon de tuyau souple (9), et en ce que dans le cas de l'actionnement de la soupape anti-retour, du liquide s'écoule dans le corps gonflable (10), sous l'effet de la pression dans le réservoir (8), en produisant l'obturation du passage d'écoulement dans le tronçon de tuyau souple (9).

8. Dispositif selon la revendication 7, caractérisé en ce que la quantité de liquide qui a été remplie dans le système constitué par le réservoir (8), le corps gonflable (10), le mécanisme de pompe (5) et les tubulures de liaison (4, 7), est juste suffisante pour amener le corps gonflable (10) dans la position de fermeture.
